# FASCICULE DE BREVET EUROPEEN

(11) **EP 0 165 830 B1**
(45) Date de publication et mention de la délivrance du brevet: **25.08.1993**
(21) Numéro de dépôt: 85400845.5
(22) Date de dépôt: 29.04.1985
(51) Int. Cl.: G01N 33/569, G01N 33/577, C12N 9/12

(54) **Anticorps monoclonaux anticytomégalovirus et procédés pour le diagnostic in vitro des infections par les cytomégalovirus humains et d'une protéine-kinase inductible par les cytomégalovirus et susceptible d'être reconnue par les susdits anticorps monoclonaux**
Verfahren zur Diagnose in vitro von menschlichen Cytomegalovirusinfektionen und Anwendung von monoklonalen Antikörpern gegen Cytomegaloviren, die mit einer cytomegalovirusinduzierten Proteinkinase reagieren
Monoclonal antibodies to cytomegalovirus and methods for the diagnosis in vitro of infections by human cytomegaloviruses, using monoclonal antibodies which react with a cytomegalovirus-induced protein kinase

(30) Priorité: 27.04.1984 FR 8406769
(43) Date de publication de la demande: 27.12.1985
(73) Titulaire: INSTITUT PASTEUR, 75724 Paris Cédex 15 (FR); CENTRE NATIONAL DE LA RECHERCHE SCIENTIFIQUE, 75007 Paris (FR); INSTITUT NATIONAL DE LA SANTE ET DE LA RECHERCHE MEDICALE (INSERM), F-75013 Paris (FR)
(72) Inventeur: Horaud, Florian, F-92190 Meudon (FR); Michelson, Susan, F-78590 Noisy Le Roi (FR); Barzu, Octavian, F-91300 Massy (FR); Boue, André, F-78210 Saint-Cyr L'Ecole (FR); Amadei, Claire, F-92400 Courbevoie (FR)
(74) Mandataire: Gutmann, Ernest

(56) Documents cités:
- CHEMICAL ABSTRACTS, vol. 101, no. 13, 24 septembre 1984, page 257, no. 106184g, Columbus, Ohio, US; S. MICHELSON et al.: "Properties of a human cytomegalovirus-induced protein kinase", & VIROLOGY 1984, 134(2), 259-68
- CHEMICAL ABSTRACTS, vol. 96, no. 9, 1 mars 1982, page 237, no. 64720z, Columbus, Ohio, US; E.C. MAR et al.: "Human cytomegalovirus-associated DNA polymerase and protein kinase activities", & J.GEN. VIROL. 1981, 57(1), 149-56
- ANNALES DE VIROLOGIE, vol. 134, no. 2, 1983, pages 165-180, Paris, FR; C. AMADEI et al.: "Kinetic study of the development and localization of human cytomegalovirus-induced antigens using monoclonal antibodies"
- INFECTION AND IMMUNITY, vol. 38, no. 1, octobre 1982, pages 273-281, American Society for Microbiology; L.C. GOLDSTEIN et al.: "Monoclonal antibodies to cytomegalovirus: Rapid identification of clinical isolates and preliminary use in diagnosis of cytomegalovirus pneumonia"
- JOURNAL OF CLINICAL MICROBIOLOGY, vol. 18, no. 2, août 1983, pages 331-343, American Society for Microbiology; K.S. KIM et al.: "Production and characterization of monoclonal antibodies specific for a glycosylated polypeptide of human cytomegalovirus"

## Description

L'invention est relative à des anticorps monoclonaux anticytomégalovirus humains (HCMV) et à un procédé pour le diagnostic in vitro des infections induites par les cytomégalovirus humains. Elle concerne plus particulièrement des anticorps de ce type qui sont capables de simultanément détecter les cytomégalovirus (CMV) humains, les cellules humaines infectées par le CMV et un polypeptide, notamment une protéine induite par les HCMV et ayant une activité de protéine-kinase.

L'invention concerne encore le susdit polypeptide ayant la susdite activité de protéine-kinase.

Il est connu que le CMV est la cause d'infections cliniques nombreuses s'étageant entre des manifestations infectieuses bénignes et des manifestations congénitales, par exemple des malformations, particulièrement sévères. Le CMV a également été reconnu comme cause de morbidité et mortalité chez les personnes ayant subi des transplantations d'organes, par exemple de rein. Le dépistage du CMV est un problème qui se pose donc de façon particulièrement aiguë. En outre la reconnaissance et l'isolement de polypeptides ou protéines vaccinants ou susceptibles d'être rendus vaccinants à l'égard du CMV ouvrirait des possibilités sérieuses de prévention desdites complications contre lesquelles on ne dispose à l'heure actuelle que de peu de moyens de traitement.

Divers auteurs ont déjà porté leur attention sur les techniques de diagnostic qui pourraient être basées sur l'utilisation d'anticorps monoclonaux à l'égard du cytomégalovirus humain. Les anticorps monoclonaux obtenus jusqu'à ce jour n'apportaient pas encore la solution attendue pour réaliser un diagnostic à la fois facile et fiable de l'infection aux cytomégalovirus.

En effet, il est connu que le CMV (membre de la famille des virus de l'herpès) induit dans les cellules infectées un nombre considérable de polypeptides, à divers stades du processus infectieux et à différents niveaux des cellules infectées. L.N. PEREIRA et Coll. ("Infection and Immunity", juin 1982, p. 924-932) rapportent qu'ils ont produit un grand nombre d'hybridomes secréteurs d'anticorps monoclonaux capables de reconnaître chez des cellules préalablement infectées et non fixées des polypeptides ou glycoprotéines induites par le CMV dans des cultures de ces cellules. Les différences observées par PEREIRA et Coll. au niveau des comportements relatifs des différents anticorps vis-à-vis du virus lui-même, des cellules infectées et des protéines, glycoprotéines ou polypeptides susceptibles d'être isolés à partir d'extraits de ces cellules, les ont conduits à formuler l'hypothèse, que la conformation des glycoprotéines virales reconnues par certains de ces anticorps à la surface membranaire des cellules infectées par les CMV, pourrait être différente de la conformation de l'enveloppe du virion. L'utilisation de certains des anticorps isolés pour réaliser des opérations de diagnostic du genre sus-indiqué a été évoquée par ces auteurs. Il résulte cependant de ce qui précède et des résultats expérimentaux fournis que les anticorps monoclonaux jugés les plus intéressants étaient également capables de précipiter à la fois plusieurs polypeptides. L'utilisation de ces anticorps pour reconnaître des déterminants antigéniques ou épitopes spécifiques ne pouvait donc être envisagée en raison du caractère non discriminant de la reconnaissance.

L.C. GOLDSTEIN et Coll. ("Infection and Immunity", octobre 1982, p. 273-281) ont également décrit un certain nombre d'anticorps monoclonaux susceptibles de reconnaître certains polypeptides localisés dans les noyaux ou dans des inclusions cytoplasmiques de cellules infectées par le CMV. Ces anticorps monoclonaux avaient été sélectionnés par leur capacité à réagir avec des cellules infectées par le CMV, et préalablement fixées au méthanol absolu et séchées à l'air. Il ne résulte cependant pas de l'article que ces anticorps monoclonaux étaient aptes à reconnaître des cellules infectées encore intactes.

Enfin C. AMADEI et Coll. (Ann. Virol. (Institut Pasteur) 1983, 134 E, 165-180) décrivent une batterie de 24 anticorps monoclonaux dirigés contre le cytomégalovirus humain. Le plus grand nombre d'anticorps monoclonaux obtenus tient vraisemblablement aux techniques qui ont été utilisées pour les détecter. En particulier, la détection a mis en jeu des techniques immunofluorescentes de détection des anticorps retenus sur des cellules préalablement infectées qui avaient été fixées à l'acétone et séchées à l'air. Les mêmes anticorps monoclonaux se sont révélés pratiquement dépourvus d'activité à l'égard de cellules obtenues à partir de lignées cellulaires identiques préalablement infectées par le même virus, lorsque ces cellules infectées avaient auparavant été fixées par le méthanol. Peut-être faut-il trouver dans cette observation la cause du faible nombre d'hybridomes secrétant des anticorps monoclonaux sélectifs contre des cellules infectées au HCMV, qui avaient été isolés par L.C. GOLDSTEIN et Coll..

Parmi ces anticorps monoclonaux certains se sont révélés présenter des propriétés neutralisantes à l'égard de plusieurs souches de virus. Ces anticorps monoclonaux ont d'ailleurs fait l'objet d'une demande de brevet français n° 83 05384 déposée le 31 mars 1983. Ces anticorps monoclonaux particuliers ont été retenus pour la réalisation d'essais de diagnostic in vitro d'une infection aux HCMV. Mais la demande de brevet et pas davantage l'article susmentionné n'ont pas reconnu parmi les différents hybridomes décrits, celui qui s'est avéré par la suite secréter des anticorps monoclonaux particulièrement efficaces pour des opérations de diagnostic et aptes à permettre l'isolement d'un polypeptide inductible par les HCMV et doué de propriétés biologiques permettant des opérations de diagnostic particulièrement raffinées.

L'invention a pour but de fournir un procédé de diagnostic mettant en oeuvre des anticorps monoclonaux résultant d'une sélection parmi l'ensemble de ceux qui ont été décrits dans l'état de la technique. L'utilisation de ces anticorps sélectionnés dans des tests de diagnostic in vitro est à la fois aisée et sure. Ils reconnaissent une protéine induite par les HCMV dans des cellules infectées encore intactes dans des essais d'immunofluorescence. Ces anticorps sont susceptibles de fournir des réponses positives pendant la quasi-totalité du cycle infectieux. Le diagnostic peut être affiné et confirmé dans des essais de dosage de l'activité enzymatique. Les cellules infectées produisent des polypeptides, protéines ou glycoprotéines qui sont induites par les HCMV. Ces polypeptides sont par ailleurs porteurs d'un épitope ou déterminant antigénique séquentiel continu. Par l'expression "porteur d'un épitope ou déterminant antigénique", il faut entendre un déterminant dont la reconnaissance par les anticorps n'est pas liée à la conformation particulière de ce site sur les polypeptides, protéines ou glycoprotéines naturels du virus ou induits par celui-ci dans les cellules qu'il infecte.

L'invention résulte de la découverte qu'ont faite les inventeurs que l'hybridome secréteur d'anticorps monoclonaux antériéurement décrit répondait à l'ensemble de ces conditions. Plus particulièrement, l'invention concerne la mise en oeuvre des anticorps monoclonaux produits par les hybridomes secréteurs concernés pour la détection in vitro d'un déterminant antigénique séquentiel porté par une protéine dont le poids moléculaire avoisine 68.000, isolable sans dégradation d'extraits de cellules infectées par une souche CMV, cette protéine possédant en outre des propriétés de protéine-kinase.

En d'autres termes, l'anticorps monoclonal anticytomégalovirus humain mis en oeuvre dans le procédé selon l'invention pour le diagnostic in vitro des infections induites par les cytomégalovirus humains et pour la détection d'une protéine-kinase inductible par les cytomégalovirus humains dans des cellules humaines, et notamment dans des lignées cellulaires humaines, peut être défini par la combinaison de ses capacités :
- à donner lieu à des réactions détectables par immunofluorescence, sur des cellules de cultures d'origine humaine, préalablement infectées avec HCMV et fixées à l'acétone ;
- à réagir avec essentiellement un seul polypeptide viral induit par les HCMV dans des cellules d'origine humaine préalablement infectéees par eux, ce polypeptide présentant un poids moléculaire de l'ordre de 68.000, porteur d'un épitope séquentiel continu, ce polypeptide apparaissant dans les noyaux et diffusant ensuite au moins en partie dans le cytoplasme desdites cellules infectées, à partir duquel il peut être isolé,
- de préférence, à se fixer sur la protéine A.

L'invention concerne encore plus particulièrement, dans le cadre des susdites applications, les anticorps monoclonaux qui reconnaissent un polypeptide répondant aux caractéristiques suivantes :
- il apparaît de manière précoce dans la cellule (il est décelable 3 à 5 heures après l'adsorption du virus sur la cellule ;
- il est spécifique du CMV humain ;
- il s'accumule dans les cellules infectées par le CMV ; il persiste au moins 4 jours dans les cellules à partir du moment de son apparition (et ce en particulier dans des fibroblastes humains du poumon type MRC5) ;
- il possède l'ensemble des propriétés de polypeptides préférés, dont les caractéristiques sont indiquées plus loin.

Les anticorps monoclonaux préférés sont ceux qui ne réagissent pas avec des récepteurs non spécifiques d'IgG, et plus particulièrement encore ceux qui se caractérisent par leur capacité à détecter le susdit polypeptide dans des cellules infectées par tout HCMV, quelle qu'en soit l'origine, par exemple des souches connues sous les désignations Ad-169, Towne et Davis. Un anticorps monoclonal préféré est celui qui est produit par l'hybridome déposé à la Collection Nationale des Cultures de Micro-Organismes (C.N.C.M.) sous le n° I-289 le 27 mars 1984.

Les susdits anticorps monoclonaux sont obtenus par un procédé mettant en jeu des hybridomes secréteurs d'anticorps monoclonaux neutralisant les virus HCMV, préalablement formés entre des cellules de myélomes et des cellules spléniques d'un animal préalablement immunisé contre un HCMV, tels que la souche HCMV Ad-169, ce procédé étant plus particulièrement caractérisé en ce que :
- l'on fait réagir lesdits anticorps neutralisants avec des cellules humaines en cultures préalablement infectées avec un HCMV et préalablement fixées avec de l'acétone, puis séchées, de préférence à l'air ;
- l'on effectue une première sélection de ceux des clones qui donnent lieu à une réaction de fixation, détectable par immunofluorescence sur lesdites cellules humaines après fixation de ces dernières à l'acétone et
- le cas échéant, on effectue une seconde sélection de ceux des anticorps monoclonaux retenus à l'issue de la première sélection, qui réagissent sélectivement avec une protéine virale apparemment induite dans les noyaux desdites cellules infectées et qui diffusent ensuite dans le cytoplasme cellulaire à partir duquel elle peut, le cas échéant, être séparée, cette protéine présentant un poids moléculaire de l'ordre de 68.000, cette protéine possédant en outre une activité de protéine-kinase et
- l'on récupère l'anticorps sélectionné à partir des produits de réaction obtenus.

La récupération de l'anticorps monoclonal peut être réalisée de toute façon en soi connue, par exemple par complexation préalable du produit de réaction formé avec une protéine A, notamment de Staphylococcus aureus, par dissocietion du complexe formé dans un tampon ionique adéquat.

L'isolement au moins partiel du polypeptide de poids moléculaire 68.000 ayant une activité de protéinekinase peut impliquer une séparation préalable des protéines ou polypeptides ayant des poids moléculaires différents, également contenus dans les extraits cytoplasmiques susceptibles d'être obtenus à partir des cellules infectées.

En ce qui concerne plus particulièrement les cellules humaines utilisables pour la production du susdit polypeptide, reconnu par les anticorps monoclonaux produits par les hybridomes dont question plus haut, on peut avoir recours à tout type de cellule susceptible de supporter la réplication du HCMV. Des cellules dont l'utilisation est avantageuse sont constituées par des fibroblastes humains de poumon du type MRC-5.

L'invention concerne également les polypeptides inductibles par les HCMV dans des cellules infectées, ayant un poids moléculaire pouvant atteindre une valeur de l'ordre de 68.000, une activité de protéine-kinase et portant un épitope séquentiel, ces polypopeptides pouvant être obtenus à partir d'une culture de cellules humaines, notamment de fibroblastes humains de poumon, préalablement infectés avec une culture de HCMV, notamment de la souche Ad-169. Ce polypeptide peut être isolé du cytoplasme des cellules infectées. Mais l'invention concerne aussi tout polypeptide de poids moléculaire plus faible, contenant également l'épitope séquentiel reconnu par les susdits anticorps monoclonaux. Il apparaîtra clairement au spécialiste qu'à partir de l'instant où l'on dispose de l'anticorps monoclonal selon l'invention, il devient possible d'envisager l'isolement à partir du susdit polypeptide ayant le poids moléculaire susdit d'environ 68.000, de séquences peptidiques plus petites contenant le même déterminant antigénique, en ayant recours à des techniques en soi connues de découpage du peptide initial intact par des enzymes susceptibles de le couper en des sites spécifiques. A titre d'exemple de telles protéines, on peut mentionner l'enzyme de Staphylococcus Aureus V8, l'alpha-chymotrypsine, la "protéase de glande sous-maxillaire de souris" (mouse submaxillary gland protease) commercialisée par la société BOEHRINGER, la collagénase de Vibrio alginoluticus chemovar iophagus qui reconnaît spécifiquement les dipeptides Gly-Pro et Gly-Ala.

Il devient alors possible, à partir des peptides fragmentés de façon contrôlée à l'aide de telles enzymes de détecter ceux qui contiennent le site antigénique par réaction avec l'anticorps monoclonal et qui, le cas échéant, conservent également les susdites activités de protéine-kinase.

Des caractéristiques supplémentaires des polypeptides préférés selon l'invention sont les suivantes :
- ils possèdent une activité protéine-kinase de type caséine-kinase II ; en particulier ils sont aptes à transférer du phosphore à partir de l'ATP à la caséine, et également à d'autres substrats, tels que la phosvitine, la glycogène-synthétase, des histones et des alpha-phosphorylases.
- l'activité de protéine-kinase est inhibée par la quercitine ;
- ce polypeptide est capable de s'autophosphoryler in vitro ;
- il est thermosensible (perte d'activité à -70°C et à 100°C).

L'invention concerne encore plus particulièrement un procédé de diagnostic in vitro mettant en oeuvre les susdits anticorps monoclonaux, ce procédé comportant la mise en contact desdits anticorps monoclonaux avec les cellules dont est recherché le caractère infecté ou non et la détection, de préférence par immunofluorescence sur les cellules traitées, sans destruction de celles-ci, du susdit polypeptide. Des conditions préférées dans lesquelles le test peut être mis en oeuvre seront indiquées plus loin.

La mise en oeuvre de ces anticorps monoclonaux particuliers dans des essais de diagnostic est particulièrement avantageuse en ce qu'elle peut être réalisée très rapidement, dès après l'infection. Il a en effet été vu que la protéine induite apparaissait de 3 à 5 heures après l'infection et persistait pendant toute la durée du cycle infectieux. Cette propriété distingue tout particulièrement cet anticorps monoclonal des autres anticorps monoclonaux. Il est particulièrement utile dans des essais de diagnostic, tant il est vrai que dans la pratique l'instant du déclenchement de l'infection est en général ignoré, surtout lorsque les essais sont réalisés sur des cellules obtenues à partir de biopsies tissulaires ou autres prélèvements cellulaires sur des patients soupçonnés d'être porteurs d'une infection aux cytomégalovirus.

Les anticorps monoclonaux mis en oeuvre dans le cadre de l'invention peuvent également être à la base de ce qui peut être considéré comme un procédé de purification d'un polypeptide protéine ou glycoprotéine, ou encore d'un fragment de ces derniers, contenant un déterminant antigénique séquentiel du HCMV et/ou possédant des activités de protéine-kinase semblable, à partir d'extraits ou de lysats de cultures cellulaires humaines, préalablement infectées par HCMV. Dans une forme préférée de ce procédé, on aura recours à un anticorps monoclonal du type de ceux qui ont été définis plus haut, fixé au préalable, par exemple au bromure de cyanogène sur un support solide, tel que le réseau d'agarose à réticulation tri-dimensionnelle, commercialisé sous la marque SEPHAROSE par la société suédoise PHARMACIA AG.

Le caractère séquentiel continu du déterminant antigénique des polypeptides selon l'invention se déduit de ce que le polypeptide le portant peut toujours être isolé à partir de l'extrait cytoplasmique de cellules infectées, même lorsque l'opération de séparation est effectuée en présence de détergents puissants comme le dodécylsulfate de sodium (SDS), le déoxycholate de sodium, ou du détergent commercialisé sous la marque TRITON X-100, avec ou sans bêta-mercapto-éthanol. Les agents susmentionnés sont en effet connus pour leur capacité à "défaire" ou à "déplier" une protéine. Tout déterminant antigénique "conformationnel" cesserait alors d'être reconnu par l'anticorps monoclonal, et ce surtout dans l'hypothèse où le "site conformationnel" mettait en jeu des séquences d'acides aminés dont le voisinage dans la protéine naturelle ne résultait que du rapporchement de séquences non directement liées entre elles, du fait de la conformation initiale de ladite protéine. Il résulte de ce qui précède que cette protéine ou polypeptide porteur dudit site antigénique est vraisemblablement également immunogène et par conséquent apte à induire la production in vivo d'anticorps neutralisant le virus lui-même.

Si les anticorps selon l'invention permettent de détecter spécifiquement la protéine de poids moléculaire d'environ 68.000 au sein d'un extrait cellulaire, le cas échéant protégé contre les dégradations enzymatiques, à l'exclusion de tout autre polypeptide ou protéine ayant des poids moléculaires distincts, il en résulte que ce polypeptide ou protéine pourra lui-même également être utilisé pour réaliser la sélection et l'isolement d'anticorps monoclonaux susceptibles d'être produits par des hybridomes obtenus par des fusions cellulaires mettant en jeu initialement d'autres types de myélomes, d'une part, et d'autres HCMV pour l'immunisation des cellules spléniques destinées à être fusionnées avec lesdites cellules de myélome, d'autre part.

Des caractéristiques supplémentaires de l'invention apparaîtront encore au cours de la description qui suit d'exemples de production d'hybridomes et d'isolement de ceux d'entre eux qui sont aptes à produire des anticorps conformes à l'invention.

Les techniques qui ont été mises en jeu dans la réalisation de ces exemples ont été réalisées selon les modes opératoires suivants :

### 1) Préparation des hybridomes

Des souris sont immunisées par injection intrapéritonéale d'une suspension de cellules MRC-5 humaines infectées et décongelées. Des cellules avaient été congelées 5 jours après leur infection avec une souche de HCMV Ad-169. Une injection de rappel est administrée à ces souris 3 ou 4 semaines plus tard. Les souris sont sacrifiées et les rates récupérées en vue de leur fusion cellulaire 3 jours après l'injection de rappel. Les cellules spléniques sont fusionnées avec des cellules de myélome SP²/OAg 14 (souche de SCHULMANN et Coll., "Nature", 1978, 276, 269/270) et les hybridomes formés sont sélectionnés dans un milieu RPMI contenant de l'hypoxanthine (5 mM) et de l'azasérine (1 mM) selon la technique décrite par R. CRAINIC et Coll., "Develop. Biol. Standard.", 1982, 50, 229-234, en rapport avec la production d'hybridomes secréteurs d'anticorps monoclonaux contre le virus de la poliomyélite.

### 2) Sélection des hybridomes positifs

Les surnageants des cultures d'hybridomes ainsi retenus ont été sélectionnés pour leur capacité à secréter des anticorps spécifiques contre HCMV par immunofluorescence indirecte, à l'aide de préparations d'antigènes tardifs. Une série d'hybridomes positifs est clonée par la méthode des dilutions limites et les surnageants de ces clones sont ensuite testés de la même façon par fluorescence indirecte, selon la méthode décrite dans l'article susmentionné de C. AMADEI et Coll. On sélectionne alors ceux des hybridomes qui secrétent des anticorps monoclonaux ayant des caractéristiques semblables à celles des anticorps monoclonaux F6b de C. AMADEI et Coll. c'est l'une de ces souches qui a été déposée à la C.N.C.M. sous le n'I-289.

### 3) Marquage des anticorps monoclonaux

Les clones (2 x 10⁶ cellules) sont marques soit avec le ³⁵S-méthionine, soit avec la ³H-méthionine, également selon la technique décrite par C. AMADEI et Coll.

### 4) Détection de la fixation sur la protéine A

Elle est là encore réalisée selon la méthode décrite dans l'article d'AMADEI et Coll..

Les essais mentionnés sous les paragraphes 3) et 4) sont plus particulièrement destinés à la reconnaissance de la capacité des anticorps monoclonaux à produire une réaction AA décelable par immunofluorescence avec des cellules humaines en cultures préalablement infectées avec HCMV, et en outre à être précipités par la protéine A.

S'agissant de détecter parmi ces anticorps monoclonaux ceux qui sont en outre capables de ne réagir qu'avec un seul polypeptide de poids moléculaire de l'ordre de 68.000, on aura recours de préférence au marquage radioactif ou analogue préalable des cellules humaines préalablement infectées et dont seront ultérieurement récupérées les extraits cellulaires. Ce sont ces extraits cellulaires que l'on fera réagir ensuite avec les anticorps monoclonaux non marqués à sélectionner.

### 5) Extraction de l'antigène

Des cellules infectées ou non infectées sont lavées deux fois in situ avec une solution saline tamponnée au phosphate (PBS) contenant des ions calcium et magnésium (PBS complet), puis récupérées par raclage dans du PBS complet contenant du fluorure de phényl-méthylsulfonyle 10⁻⁴ M (PMSF) et du diisopropylfluorophosphate 10⁻⁴ (DFP), les cellules étant enfin soumises à extraction dans une solution ayant une teneur élevée en sel et à pH élevé contenant 0,5 % du détergent commercialisé sous la désignation NP-40, selon la technique de MICHELSON et Coll. (1979) J. Virol. 32, 259-267. Dans certains cas, les noyaux ont été séparés du cytoplasme après gonflage des cellules dans un tampon hypotonique, addition de NP-40 (concentration finale 0,5 %) et soumission de la suspension de cellules à l'action d'un piston plongeur pour casser les cellules (selon la technique de MICHELSON et Coll.). La suspension est ensuite centrifugée à 800 g pendant 5 minutes à 4°C. Les noyaux sont alors retenus dans le culot formé et le surnageant constituant la fraction cytoplasmique est alors récupéré. Les noyaux ont été soumis au même traitement d'extraction que mentionné plus haut.

Tous les extraits ont été centrifugés à 15.000 g pendant 15-30 minutes à 4°C et congelés à -70°C, jusqu'au moment de l'utilisation. Pour les essais de mesure de l'activité phosphotransférase, on procède à l'immunoprécipitation immédiate de l'antigène recherché dans les conditions indiquées ci-après. L'antigène peut être conservé à l'état lié à Staphylococcus aureus.

### 6) Immunoprécipitation

Le précipitation est réalisée en mettant en oeuvre 5 microlitres de fluide d'ascite par 200 microgrammes de protéine extraite. L'antigène et les anticorps monoclonaux (F6b) sont incubés sous agitation pendant 1,5 heure à température ambiante. On ajoute la protéine A sous le forme d'une suspension à 10 % d'une souche de S. aureus sérotype I de Cowan inactivée à la chaleur et fixée par le formaldéhyde (50 microlitres/5 microlitres de fluide d'ascite). Les complexes antigène-anticorps sont levés trois fois dans le milieu de NETS (KESSLER S.W. (1975) J. Immunol. 115, 1617-1624), puis conservés dans ce tampon sous la forme d'une suspension à 10 % à 4°C. Le complexe peut aussi être dissocié dans un tampon d'électrophorèse par chauffage pendant 5 minutes à 100°C. L'immunoprécipitat a été analysé sur le gel d'électrophorèse sodium-dodécyl-sulfate-polyacrylamide (SDS-PAGE à 10 %). La résolution des protéines a été réalisée par migration différentielle sous une intensité de 30-35 mA. Le cas échéant, la fluorographie a été réalisée selon la méthode de BONNAR et Coll. (1974) Eur. J. Biochem. 46, 83-88, avec la modification consistant en l'utilisation d'un seul bain à base de diméthylsulfoxide (DMSO) pendant 15 minutes à 37°C et d'un seul bain de DMSO + 20 % de 2,5-diphényloxazole (PPO) pendant 30 minutes à 37°C.

### 7) Marquage radioactif des cellules

Des cellules infectées sont sélectionnées 1 heure postérieurement à l'infection et privées de méthionine pendant 15 minutes. L'absorption avec le virus est ensuite conduite dans un milieu contenant 10 microcuries/millilitre de ³⁵S-méthionine. Pour les autres échantillons de cellules infectées, le virus a été absorbé pendant 1 heure et le milieu pour la croissance a été ajouté. Toutes les cellules ont été privées de méthionine pendant 15 minutes avant d'être marquées pendant 2 heures avant leur prélèvement à des instants respectifs de 3, 9, 24, 48, 72 et 96 heures après l'infection. Les cellules non infectées ont été marquées de la même façon. Le milieu de marquage consistait en un milieu essentiel minimum de Eagle dépourvu de méthionine contenant 10 microcuries/millitre de ³⁵S-méthionine (AMERSHAM, activité spécifique de 1300 curies/mMole). Le marquage des phosphoprotéines a été réalisé pendant 3 heures dans un milieu modifié de DULBECCO dépourvu de phosphate contenant 100 microcuries/millilitre de ³²P-orthophosphate.

### 8) Mise en évidence de l'activité de protéine-kinase

Sauf lorsque spécifié autrement, l'essai pour l'activité de protéine-kinase est réalisé en utilisant 50 microlitres d'enzyme liée à des bactéries dans un milieu (volume final de 100 microlitres) contenant du tampon au phosphate de magnésium 22 mM à pH 6,8 KPi), du sulfate de magnésium MgSO₄ 5 mM, de l'EDTA 0,15 mM, de l'ATP 0,1 mM, 3 microcuries de ³²P-ATP et 1 milligramme par millilitre de caséine. La caséine n'est pas présente dans les essais d'analyse des immunoprécipitats dans le gel SDS-PAGE. Les immunoprécipitats sont alors lavés comme indiqué plus haut, remis en suspension dans le mélange de réaction, et incubés pendant 30 minutes à 30°C. La réaction est stoppée avec 10 microlitres de solution d'EDTA 0,3 M. Les immunoprécipitats sont séparés par centrifugation à 5000 g pendant 4 minutes à 4°C. Les surnageants sont précipités avec une solution d'acide trichloracétique à 5 % (TCA). Les précipitats sont dissous dans une solution de soude 1 M, puis reprécipités par le TCA, lavés par filtration sur des filtres commercialisés sous le désignation WHATMAN G.F./C. Après lavage supplémentaire dans l'éthanol, les filtres sont séchés et les impulsions comptées dans un liquide scintillateur PACKARD. Les immunoprécipitats destinés à l'analyse sur gel sont lavés trois fois dans le tampon KPi, remis en suspension dans le tampon de l'électrophorèse et analysés dans le gel SDS/PAGE à 10 % dans les conditions sus-indiquées.

### 9) Estimation du coefficient de sédimentation

L'antigène marqué à la ³⁵S-méthionine a été préparé et extrait comme indiqué plus haut à partir de cellules infectées pendant 120 heures. L'antigène est ensuite déposé à la partie supérieure d'un gradient de sucrose 5-25 % formé dans du PBS et soumis à centrifugation dans un rotor BECKMAN SW-41 à 35.000 révolutions par minute pendant 17,25 heures à 4°C. Des fractions ont été collectées. Chaque fraction a été immunoprécipitée avec l'anticorps monoclonal F6b, puis analysée sur SDS-PAGE.

### 10) Electrophorèse des aminoacides marqués au ³²P

Après la réaction de protéine-kinase mettant en oeuvre des immunoprécipitats obtenus en l'absence de caséine, le produit marqué au ³²P est détaché avec une solution de NH₄HCO₃ à 0,1 % contenant 0,1 % de SDS. L'échantillon est séché sous vide, remis en suspension dans HCl 6M et hydrolysé pendant la nuit A 110°C. Après évaporation de HCl, les préparations sont remises en suspension dans le tampon d'électrophorèse. Les phosphoaminoacides sont séparés à pH 3,5 (dans un milieu formé à partir de 50 millilitres d'acide acétique et de 5 millilitres de pyridine par litre) pendant 45 minutes sous 1000 V sur des plaques minces revêtues de cellulose (commercialisées par la société MACHEREY-NAGEL & Co). Des étalons ont été colorés avec le réactif cadmiumninhydrine (décrits par DRICKAMER et Coll., J. Biol. Chem. (1982) 257, 15156-15161) et les plaques ont été exposées à une pellicule Kodak X-Omat pendant une semaine.

Le polypeptide immunoprécipité par l'anticorps monoclonal F6b présente une masse moléculaire d'environ 68.000 (p68). Il est immunoprécipitable à partir des noyaux des cellules infectées dès la troisième heure après l'infection. Il peut être immunoprécipité également à partir des extraits cytoplasmiques 24 heures après l'infection. La teneur en ce polypeptide vis-à-vis de l'ensemble des protéines extraites, 96 heures après l'infection, peut atteindre 0,5 % (estimation sur la base du pourcentage de radioactivité contenu dans l'immunoprécipitat récupéré). Ce polypeptide présente un coefficient de sédimentation dans l'eau à 20° de 6,9 (par comparaison à des marqueurs consistant en catalase et en sérum albumine bovine).

Le poids moléculaire de p68 a été apprécié par rapport aux distances demigration dans un même système électrophorétique, des quatre substances de poids moléculaires connus qui suivent :
- phosphorylase A (94.000) ;
- sérumalbumine bovine (68.000) ;
- fumarase (49.000) ;
- aldolase (40.000).

Ce polypeptide témoigne des capacités de phosphorylation in vitro qui ont été indiquées plus haut. La réaction de protéine-kinase avec la caséine utilisée en tant qu'accepteur se fait de façon optimale à pH 6-6,5. Elle décline rapidement dans des milieux à pH plus acides ou plus alcalins. La réaction est dépendante de la présence d'ions Mg²⁺. Ces réactions ne sont pas cAMP-dépendantes. La réaction de transfert de phosphate ne s'opère pas en présence de manganèse à pH élevé. Ce polypeptide (ou cette enzyme) est stable. Il peut être conservé à l'état conjugué avec S. aureus, sans perte d'activité après conservation pendant 6 semaines à 4°C. Le complexetion avec les anticorps monoclonaux F6b n'entraîne pas le perte d'activité.

Cette enzyme est capable de s'autophosphoryler, celle-ci intervenant au niveau de résidus aminoacyles constitués par la thréonine et la sérine.

Des polypeptides ayant des poids moléculaires de l'ordre de 68.000 et présentant les mêmes caractéristiques que celles qui ont été indiquées plus haut sont induits dans des cellules humaines infectées, notamment des fibroblastes de poumon, par d'autres souches de cytomégalovirus, particulièrement les souches humaines TOWNE et DAVIS. Par contre des souches de cytomégalovirus spécifiques du singe (souche COLBURN et souche SGC) n'induisent pas la production d'une protéine de poids moléculaire 68.000 reconnaissable par les anticorps monoclonaux secrétés par la souche F6b.

L'invention concerne donc plus particulièrement la sélection des souches productrices d'anticorps monoclonaux ayant les caractéristiques des anticorps monoclonaux F6b.

La mise en oeuvre de cet anticorps monoclonal permet en effet des diagnostics sûrs in vitro d'une infection virale aux HCMV. Il a déjà été indiqué que cette détection pouvait se faire pratiquement à tout moment du cycle infectieux, notamment par des réactions d'immunofluorescence. Cet anticorps monoclonal présente encore le grand avantage que le diagnostic réalisé par immunofluorescence peut être confirmé, par la capacité du même anticorps à former des complexes avec une protéine induite par les cytomégalovirus humains, protéine dont la présence peut ensuite être décelée par des activités de protéine-kinase. Celles-ci peuvent être détectées avec un niveau de précision élevé. Enfin l'utilisation d'anticorps monoclonaux du type sus-indqué peut être mis en oeuvre dans des essais de dosage quantitatif du niveau d'infection des cellules humaines atteintes.

L'invention concerne également le polypeptide lui-même, en tant que réactif biologique de grande valeur, par exemple dans des essais d'identification de virus, dès lors que leur classification au sein des cytomégalovirus n'apparaît pas certaine a priori. A l'inverse, il peut être utilisé pour la sélection des anticorps monoclonaux les plus efficaces pour la constitution de "kits" de diagnostic.

L'invention concerne encore des nécessaires ou "kits" pour réaliser des diagnostics in vitro comme décrits plus haut. Un tel nécessaire pourra notamment comprendre :
- au moins une micro-plaque,
- une préparation contenant des anticorps monoclonaux appropriés au diagnostic,
- des extraits cellulaires témoins (obtenus à partir de cellules humaines, notamment du type MRC-5 humaines, ces cellules ayant ou non été préalablement infectées avec un HCMV),
- des anticorps marqués (marqueur radioactif, enzymatique, fluorescent ou autre) dirigés contre les immunoglobulines du sang humain,
- les tampons appropriés à la réalisation des différentes opérations et à l'examen final des produits ayant réagi, au microscope à fluorescence.

Un tel nécessaire sera par exemple mis en oeuvre de la façon suivante :
- un extrait cellulaire provenant d'une biopsie cellulaire à examiner est déposé dans une ou plusieurs cupules de la plaque de titration et adsorbé à leur surface ;
- on introduit dans les cupules ou puits de la micro-plaque des doses de la préparation d'anticorps monoclnal ;
- le plaque est mise à incuber à le température appropriée ;
- le microplaque est ensuite efficacement levée ;
- on introduit ensuite dans les puits de le microplaque des anticorps marqués dirigés contre des immunoglobulines sanguines, donc contre les anticorps monoclonaux et on incube à nouveau ;
- on détecte le marqueur (par exemple par action sur le substrat approprié, lorsque le marqueur est constitué par une enzyme),
   l'ensemble de ces opérations étant également réalisé à l'égard d'extraits cellulaires témoins, et ce à des fins comparatives.

Avantageusement, on peut également réaliser des essais de détection et de dosage quantitatifs, par exemple par passage d'un extrait cellulaire obtenu dans des conditions par exemple du genre de celles qui ont été envisagées plus haut, sur une colonne de chromatographie d'affinité comportant les anticorps monoclonaux fixés sur gel d'agarose, par exemple comme il a été indiqué plus haut, la dissociation du complexe éventuellement formé entre les anticorps monoclonaux utilisés et le polypeptide spécifiquement reconnu par les derniers, et le dosage quantitatif du polypeptide, en mettant en oeuvre ses propriétés de protéine-kinase.

Comme il va de soi et comme il résulte d'ailleurs déjà de ce qui précède, l'invention ne se limite nullement à ceux de ses modes d'application et de réalisation qui ont été plus spécialement envisagés ; elle en embrasse au contraire toutes les variantes.

## Revendications

1. Procédé de détection de la présence d'une infection, notamment de diagnostic in vitro, par les cytomégalovirus humains dans des cellules d'origine humaine, provenant de prélèvements cellulaires, notamment de biopsies cellulaires, sur des patients soupçonnés d'être infectés par un cytomégalovirus, caractérisé par la mise en contact de ces cellules à l'état intact ou avec des extraits cellulaires obtenus à partir de ces cellules, avec un anticorps monoclonal défini par la combinaison de propriétés suivantes :
- il donne lieu à des réactions détectables par immunofluorescence, sur des cellules intactes de culture d'origine humaine, préalablement infectées avec HCMV et fixées à l'acétone ;
- il réagit essentiellement avec un seul polypeptide viral induit par les HCMV dans des cellules d'origine humaine préalablement infectées par eux, ce polypeptide présentant un poids moléculaire de l'ordre de 68.000, porteur d'un épitope séquentiel continu et ayant des propriétés de protéine-kinase-II, ce polypeptide apparaissant dans les noyaux et diffusant ensuite au moins en partie dans le cytoplasme desdites cellules infectées, à partir duquel il peut être isolé,
- de préférence, il réagit avec la protéine A.

2. Procédé selon la revendication 1, caractérisé en ce que l'on met en oeuvre un anticorps monoclonal réagissant avec un polypeptide de poids moléculaire 68.000 :
- qui apparaît de manière précoce dans la cellule (il est décelable 3 à 5 heures après l'adsorption du virus sur la cellule) :
- qui s'accumule dans les cellules infectées par le CMV et qui persiste au moins pendant 4 jours dans les cellules à partir du moment de son apparition.

3. Polypeptide inductible dans des cellules d'origine humaine par un cytomégalovirus humain et présentant les caractéristiques suivantes :
- il possède un poids moléculaire d'environ 68.000 ;
- il possède une activité protéine-kinsase de type caséine-kinase-II ;
- il est apte à transférer du phosphore à partir de l'ATP à la caséine, et également à d'autres substrats, tels que la phosvitine, la glycogène-synthétase, des histones et des alpha-phosphorylases ;
- son activité de protéine-kinase est inhibée par la quercitine ;
- il est capable de s'autophosphoryler in vitro ;
- il est thermosensible (perte d'activité à -70°C et à 100°C) ;
- il est complexé par l'anticorps monoclonal déposé à la C.N.C.M. sous le N° I-289.

4. Polypeptide selon la revendication 3, dont l'activité de protéine-kinase est dépendante de la présence d'ions Mg²⁺, mais qui n'est dépendante ni de la présence d'ions manganèse, ni de la présence cAMP.

5. Polypeptide selon l'une des revendications 3 et 4, caractérisé en ce qu'il est conjugué à de la protéine A de Staphylococcus aureus.

6. Procédé de détection dans un extrait de cellules d'un polypeptide tel que défini dans l'une quelconque des revendications 3 à 5, et induit dans ces cellules par un cytomégalovirus humain, caractérisé en ce qu'on fait réagir un extrait de ces cellules avec un anticorps monoclonal du type défini dans la revendication 1 ou dans la revendication 2 et en ce que l'on détecte le complexe formé entre l'anticorps monoclonal et le polypeptide par mesure de l'activité de protéine-kinase dudit polypeptide.

7. Polypeptide selon l'une quelconque des revendications 3 à 5, caractérisé en ce qu'il est à l'état de complexe avec un anticorps monoclonal tel que défini dans l'une quelconque des revendications 1 à 2.

8. Nécessaire de diagnostic d'une infection cellulaire par des cytomégalovirus humains caractérisé en ce qu'il comprend :
- au moins une micro-plaque,
- une préparation contenant un anticorps monoclonal du type défini dans la revendication 1 ou dans la revendication 2,
- des extraits cellulaires (témoins obtenus à partir de cellules humaines, notamment du type MRC-5 humaines, ces cellules ayant ou non été préalablement infectées avec un HCMV),
- des anticorps marqués (marqueur radioactif, enzymatique, fluorescent ou autre) dirigés contre les immunoglobines du sang humain,
- les tampons appropriés à la réalisation des différentes opérations et à l'examen final des produits ayant réagi, au microscope à fluorescence.

## Claims

1. Procedure for the detection of the presence of an infection, in particular by in vitro diagnosis, due to the presence of human cytomegaloviruses in cells of human origin, obtained from cell samples, in particular cell biopsies, taken from a patients suspected to be infected by a cytomegalovirus, characterized by placing these cells in the intact state or cell extracts obtained from these cells in contact with a monoclonal antibody defined by the combination of the following properties :
- it gives rise to reactions with a culture of intact cells of human origin, previously infected with HCMV and fixed with acetone, which can be detected by immunofluorescence;
- it reacts essentially with a single viral polypeptide induced by the HCMV in cells of human origin previously infected by them; this polypeptide has a molecular weight of the order of 68,000, bears a continuous sequential epitope and has the properties of protein kinase II, this polypeptide appearing in the nuclei and then diffusing at least in part into the cytoplasm of the said infected cells, from which it can be isolated,
- preferably, it reacts with protein A.

2. Procedure according to Claim 1, characterized in that a monoclonal antibody is used which reacts with a polypeptide having a molecular weight of 68,000 :
- which appears early in the cell (it can be detected 3 to 5 hours after the adsorption of the virus to the cell) :
- which accumulates in the cells infected by the CMV and which persists for at least 4 days in the cells after its initial appearance.

3. Polypeptide which can be induced in cells of human origin by a human cytomegalovirus and which exhibits the following properties :
- it possesses a molecular weight of about 68,000 ;
- it possesses a protein kinase activity of the casein kinase II type ;
- it is capable of transferring phosphate from ATP to casein, and also to other substrates, such as phosvitin, glycogen synthetase, histones and alpha phosphorylases ;
- its protein kinase activity is inhibited by quercitin ;
- it is capable of autophosphorylation in vitro ;
- it is temperature-sensitive (loss of activity at -70°C and at 100°C) ;
- it forms a complex with the monoclonal antibody deposited with the C.N.C.M. under the No. I-289.

4. Polypeptide according to Claim 3, the protein kinase activity of which is dependent on the presence of Mg²⁺, but is not dependent on the presence of either manganese ions or cAMP.

5. Polypeptide according to one of the Claims 3 and 4, characterized in that it is conjugated to protein A of Staphylococcus aureus.

6. Procedure for the detection in a cell extract of a polypeptide such as that defined in any one of the Claims 3 to 5 and which is induced in these cells by a human cytomegalovirus, characterized in that an extract of these cells is allowed to react with a monoclonal antibody of the type defined in Claim 1 or Claim 2 and in that the complex formed between the monoclonal antibody and the polypeptide is detected by measurement of the protein kinase activity of the said polypeptide.

7. Polypeptide according to any one of the Claims 3 to 5, characterized in that it is in the state of a complex with a monoclonal antibody such as defined in any one of the Claims 1 to 2.

8. Diagnostic kit for a cellular infection by human cytomegaloviruses, characterized in that it contains :
- at least one micro-plate,
- a preparation containing a monoclonal antibody of the type defined in Claim 1 or Claim 2,
- cell extracts (controls obtained from human cells, in particular of the human MRC-5 type, these cells having been infected or not infected beforehand with a HCMV),
- labelled antibodies (radioactive, enzymatic, fluorescent or other label) directed against the immunoglobulins of human blood,
- the buffers necessary for the performance of the various operations and for the final examination of the products which have reacted, by means of the fluorescence microscope.

## Patentansprüche

1. Verfahren zum Nachweisen, vor allem zur in vitro-Diagnose, einer Infektion, hervorgerufen durch menschliche Zytomegalieviren in Zellen menschlicher Herkunft, die von Zellproben, vor allem von Zellbiopsien, von Patienten stammen, die im Verdacht stehen, von einem Zytomegalievirus infiziert worden zu sein, dadurch gekennzeichnet, daß man diese Zellen im intakten Zustand oder ausgehend von diesen Zellen erhaltene Zellextrakte mit einem monoklonalen Antikörper zusammenbringt, der durch die Kombination folgender Eigenschaften definiert ist:
- er führt zu mittels Immunofluoreszenz nachweisbaren Reaktionen an intakten Zellen einer Kultur menschlicher Herkunft, die zuvor mit HCMV infiziert und an Aceton fixiert worden sind;
- er reagiert im wesentlichen mit einem einzigen viralen Polypeptid, das durch die HCMV in die zuvor von ihnen infizierten Zellen menschlicher Herkunft induziert worden ist, wobei dieses Polypeptid ein Molekulargewicht in der Größenordnung von 68 000 aufweist, ein zusammenhängendes sequentielles Epitop trägt und Eigenschaften der Proteinkinase-II besitzt, wobei dieses Polypeptid in den Kernen auftritt und dann zumindest teilweise in das Zytoplasma der infizierten Zellen diffundiert und daraus isoliert werden kann;
- er reagiert vorzugsweise mit Protein A.

2. Verfahren nach Anspruch 1, dadurch gekennzeichnet, daß man einen monoklonalen Antikörper einsetzt, der mit einem Polypeptid vom Molekulargewicht 68 000 reagiert, das:
- frühzeitig in der Zelle auftritt (es ist 3 bis 5 h nach Adsorption des Virus an der Zelle nachweisbar),
- sich in den vom CMV infizierten Zellen akkumuliert und mindestens 4 Tage bestehen bleibt, gerechnet vom Zeitpunkt seines Auftretens an.

3. Polypeptid, das in Zellen menschlicher Herkunft durch einen menschlichen Zytomegalievirus induzierbar ist und folgende Merkmale aufweist:
- es besitzt ein Molekulargewicht von etwa 68 000;
- es besitzt eine Proteinkinase-Aktivität vom Typ Caseinkinase-II;
- es kann Phosphor von der ATP zum Casein und auch zu anderen Substraten wie Phosvitin, Glykogen-Synthetase, Histone und α-Phosphorylasen übertragen;
- seine Proteinkinase-Aktivität wird durch Quercitin gehemmt;
- es kann in vitro sich selbst phosphorylieren;
- es ist Temperatur-empfindlich (Aktivitätsverlust bei -70°C und bei 100°C);
- es wird von dem bei der C.N.C.M. unter der Nr.I-289 hinterlegten monoklonalen Antikörper komplex gebunden.

4. Polypeptid nach Anspruch 3, dessen Proteinkinase-Aktivität von der Anwesenheit von Mg(II)-Ionen abhängt, aber weder von der Anwesenheit von Manganionen noch von der Anwesenheit von cAMP abhängt.

5. Polypeptid nach einem der Ansprüche 3 und 4, dadurch gekennzeichnet, daß es an das Protein A von Staphylococcus aureus konjugiert ist.

6. Verfahren zum Nachweisen in einem Zellextrakt eines Polypeptids, wie in einem der Ansprüche 3 bis 5 definiert, das in diese Zellen durch einen menschlichen Zytomegalievirus induziert worden ist, dadurch gekennzeichnet, daß man einen Extrakt dieser Zellen mit einem monoklonalen Antikörper des in Anspruch 1 oder Anspruch 2 definierten Typs reagieren läßt und daß man den zwischen dem monoklonalen Antikörper und dem Polypeptid gebildeten Komplex durch Messen der Proteinkinase-Aktivität des Polypeptids nachweist.

7. Polypeptid nach einem der Ansprüche 3 bis 5, dadurch gekennzeichnet, daß es im Zustand eines Komplexes mit einem monoklonalen Antikörper, wie in einem der Ansprüche 1 und 2 definiert, vorliegt.

8. Diagnosebesteck für eine Zellinfektion durch menschliche Zytomegalieviren, dadurch gekennzeichnet, daß es umfaßt:
- mindestens eine Mikroplatte,
- ein Präparat, das einen monoklonalen Antikörper des im Anspruch 1 oder im Anspruch 2 definierten Typs enthält,
- Zellextrakte (Vergleichs-/Bezugsextrakte, erhalten aus menschlichen Zellen, vor allem vom Typ menschliche MRC-5, wobei diese Zellen zuvor mit einem HCMV infiziert oder nicht infiziert worden sind),
- markierte Antikörper (radioaktiver, enzymatischer, fluoreszierender oder anderer Marker), die gegen die Immunoglobine des menschlichen Blutes gerichtet sind,
- geeignete Puffer für die Durchführung der verschiedenen Arbeitsgänge und die abschließende Untersuchung der Produkte, die reagiert haben, im Fluoreszenzmikroskop.
